# FASCICULE DE BREVET EUROPEEN

(11) **EP 4 448 030 B1**
(45) Date de publication et mention de la délivrance du brevet: **03.09.2025**
(21) Numéro de dépôt: 22850586.3
(22) Date de dépôt: 16.12.2022
(51) Int. Cl.: A61L 24/00

(54) **COMPOSITION ADHÉSIVE OSSEUSE**
KNOCHENKLEBERZUSAMMENSETZUNG
BONE ADHESIVE COMPOSITION

(30) Priorité: 17.12.2021 FR 2113780
(43) Date de publication de la demande: 23.10.2024
(73) Titulaire: INSERM (INSTITUT NATIONAL DE LA SANTE ET DE LA RECHERCHE MEDICALE), 75013 Paris (FR); Centre Hospitalier Universitaire de Lille, 59000 Lille (FR); Université de Lille, 59000 Lille (FR); INSTITUT NATIONAL DE RECHERCHE POUR L'AGRICULTURE, L'ALIMENTATION ET L'ENVIRONNEMENT, 75007 Paris (FR); Centre National de la Recherche Scientifique, 75016 Paris (FR); Centrale Lille Institut, 59650 Villeneuve-d'Ascq (FR)
(72) Inventeur: SCHLUND, Mathias, 59660 LILLE (FR); CHAI, Feng, 59120 LOOS (FR); LYSKAWA, Joël, 59310 COUTICHES (FR); FERRI, Joël, 59650 VILLENEUVE D'ASCQ (FR)
(74) Mandataire: Plasseraud IP
(86) Numéro de dépôt international: PCT/FR2022/052402
(87) Numéro de publication internationale: WO 2023/111485

(56) Documents cités:
- ALINA KIRILLOVA ET AL: "Bioinspired Mineral-Organic Bioresorbable Bone Adhesive", ADVANCED HEALTHCARE MATERIALS, vol. 7, no. 17, 1 September 2018 (2018-09-01), DE, pages 1800467, XP055596766, ISSN: 2192-2640, DOI: 10.1002/adhm.201800467
- LIU ZONGGUANG ET AL: "Effect of polydopamine on the biomimetic mineralization of mussel-inspired calcium phosphate cement in vitro", MATERIALS SCIENCE AND ENGINEERING C, ELSEVIER SCIENCE S.A, CH, vol. 44, 12 August 2014 (2014-08-12), pages 44 - 51, XP029050805, ISSN: 0928-4931, DOI: 10.1016/J.MSEC.2014.07.063
- SUN NAN ET AL: "Dopamine-Mediated Biomineralization of Calcium Phosphate as a Strategy to Facilely Synthesize Functionalized Hybrids", JOURNAL OF PHYSICAL CHEMISTRY LETTERS, vol. 12, no. 41, 21 October 2021 (2021-10-21), US, pages 10235 - 10241, XP055945579, ISSN: 1948-7185, DOI: 10.1021/acs.jpclett.1c02748

## Description

La présente invention concerne une composition adhésive comprenant une céramique phosphocalcique choisie parmi le tétracalcium phosphate et l'alpha-tricalcium phosphate, de la sérine phosphorylée, et de la polydopamine, son procédé de préparation et son utilisation.

### INTRODUCTION

La fracture osseuse est une pathologie très fréquente. Le traitement des fractures repose sur la réduction du foyer de fracture puis à sa contention jusqu'à consolidation. La contention peut être obtenue soit de façon orthopédique par blocage maxillo-mandibulaire au niveau facial ou par plâtre au niveau des membres, soit de façon chirurgicale par ostéosynthèse. Le traitement chirurgical, permettant une remise en fonction plus rapide, est de notre jour largement préféré au traitement orthopédique à l'exception de certaines fractures particulières et de cas pédiatriques.

La contention chirurgicale repose actuellement sur du matériel d'ostéosynthèse métallique, principalement constitué de plaques métalliques transvissées de part et d'autre du trait de fracture. Par ailleurs, les fractures ne sont pas les seules interventions où une fixation osseuse est nécessaire. Les techniques d'autogreffes osseuses pour le comblement de pertes de substances osseuses ou à visée préprothétique au niveau craniofacial nécessitent la fixation des fragments osseux pour empêcher toute mobilité du greffon. Le matériel métallique est également utilisé pour la fixation osseuse dans les ostéotomies chirurgicales (chirurgie orthognathique, ostéotomie du tibia) et les arthrodèses rachidiennes. Enfin, l'intégration d'implants métalliques dans l'os (implants dentaires, implants articulaires) nécessite un temps d'ostéo-intégration qui pourrait être réduit par l'ajout d'un adhésif. Dans les cas où un implant métallique serait nécessaire, l'adhésif pourrait assurer sa fixation.

Les cas de fractures comminutives (multi-fragmentaires) sont particulièrement complexes à traiter avec du matériel métallique en raison de la petite taille des fragments par rapport à la taille des vis. Le tiers moyen et supérieur de la face offre un bon exemple de ces fractures difficiles à traiter avec le modèle classique de plaques transvissées en raison de fractures généralement très comminutives dans un os fin et fragile. Les membres sont également sujets à ce type de fracture dans certains traumatismes à haute vélocité. Par ailleurs, les fractures intra-articulaires posent un problème puisque le matériel peut interférer avec le fonctionnement de l'articulation. Enfin, le matériel métallique ne suivant pas la croissance des os, il est peu adapté aux cas de fractures pédiatriques dans un squelette en croissance.

Un adhésif osseux ou « colle à os » offrirait une solution simple et rapide pour la prise en charge des fractures, tout particulièrement des fractures pour lesquels les systèmes actuels d'implants métalliques sont mal adaptés. Biorésorbable, il permettrait d'éviter les complications infectieuses et mécaniques (débricolage du matériel) liées aux implants métalliques et les nombreuses interventions réalisées pour retirer ce matériel, interventions liées à un risque de morbidité post-opératoire et à un poids économique certain (hospitalisation et acte chirurgical supplémentaire, arrêt de travail).

Il n'existe actuellement aucune alternative commercialisée au matériel d'ostéosynthèse métallique, comme un adhésif osseux. Le principal écueil étant l'adhésion en milieu physiologique humide. Des études et des brevets ont déjà été publiés associant la céramique phosphocalcique (CPC) et de la sérine phosphorylée (O-PhosphoSérine/OPS). Les céramiques phosphocalciques présentent une excellente biocompatibilité et des propriétés ostéo-conductrices. Elles sont utilisées en pratique clinique courante depuis de nombreuses années en tant que substitut osseux. Toutefois, le principal problème des céramiques phosphocalciques pour la fixation osseuse est leur absence d'adhésion au tissu osseux.

Inspirée par les mécanismes d'adhésion observés dans la nature, notamment les animaux marins, la sérine phosphorylée peut être adjoint à une céramique phosphocalcique pour obtenir des propriétés adhésives. La sérine phosphorylée est une molécule utilisée par le ver marin *Phragmatopoma californica* (ou *Sandcastle worm*) qui a la capacité de construire des coques de protection sous-marines en collant des minéraux selon un mécanisme de coacervation complexe. De plus, la structure de la sérine phosphorylée est proche de l'ostéopontine, ce qui lui confère un caractère ostéo-inducteur.

Des adhésifs osseux constitués de sérine phosphorylée et de céramique phosphocalcique ont déjà été étudiés avec une confirmation de l'efficacité adhésive *ex vivo* et de la biocompatibilité *in vivo* mais aucune étude n'a confirmé la capacité adhésive de cette composition *in vivo* (Bioinspired Mineral-Organic Bioresorbable Bone Adhesive, Kirillova et al., Advanced Healthcare Materials, 2018, doi: 10.1002/adhm.201800467).

Il est donc nécessaire de pouvoir disposer d'une composition adhésive préparée à partir de céramique phosphocalcique et de sérine phosphorylée ayant une capacité adhésive in vivo tout en étant biocompatible et sure.

Poursuivant ces recherches par de très nombreux travaux, les demandeurs ont trouvé qu'une composition adhésive comprenant du tétracalcium phosphate, de la sérine phosphorylée et de la polydopamine, présentait de telles caractéristiques.

D'autres caractéristiques et avantages de la présente invention apparaîtront à la lecture de la description détaillée suivante.

### RESUME DE L'INVENTION

Un premier objet de la présente invention concerne une composition adhésive comprenant :
- une céramique phosphocalcique choisie parmi le tétracalcium phosphate et l'alpha-tricalcium phosphate,
- de la sérine phosphorylée,
- de la polydopamine, et
- un solvant aqueux.

Un second objet de la présente invention concerne un kit pour préparer une composition adhésive selon l'invention, comprenant :
- une céramique phosphocalcique choisie parmi le tétracalcium phosphate et l'alpha-tricalcium phosphate de préférence le tétracalcium phosphate,
- de la sérine phosphorylée, et
- de la polydopamine.

Un troisième objet de la présente invention concerne un procédé de préparation d'une composition adhésive selon l'invention comprenant les étapes suivantes :
a) Mélanger une céramique phosphocalcique choisie parmi le tétracalcium phosphate et l'alpha-tricalcium phosphate, de la sérine phosphorylée et de la polydopamine dans un récipient,
b) Ajouter un solvant à la précédente préparation,
c) Récupérer le mélange ainsi formé.

Un dernier objet de la présente invention concerne la composition selon l'invention pour son utilisation thérapeutique *in vivo* comme adhésif osseux.

### DESCRIPTION DETAILLEE

### La composition adhésive

La composition adhésive selon l'invention comprend :
- une céramique phosphocalcique choisie parmi le tétracalcium phosphate et l'alpha-tricalcium phosphate, de préférence le tétracalcium phosphate,
- de la sérine phosphorylée,
- de la polydopamine, et
- un solvant aqueux.

La céramique phosphocalcique est composée d'un sel métallique polyvalent biocompatible qui réagit avec la sérine phosphorylée, composé phosphate organique, dans un environnement aqueux pour former des compositions dotées de puissantes propriétés adhésives.

La céramique phosphocalcique selon la présente invention est choisie parmi le tétracalcium phosphate, par exemple celui de chez Matexcel ou Hangzhou ICH Biofarm, et l'alpha-tricalcium phosphate, par exemple celui de chez Matexcel, Innotere ou Merck. De préférence, la céramique phosphocalcique est du tétracalcium phosphate.

La quantité de céramique phosphocalcique dans la composition peut varier et est de préférence comprise entre 50% et 80%, de préférence entre 60% et 70%, de manière plus préférée entre 65 et 68% en poids sec sur le poids total de la composition.

La sérine phosphorylée selon la présente invention, par exemple la O-Phospho-DL-Sérine ou la O-Phospho-L-Sérine de chez Merck, est une molécule biocompatible utilisée par le ver marin *Phragmatopoma californica* (ou Sandcastle worm) qui a la capacité de construire des coques de protection sous-marines en collant des minéraux selon un mécanisme de coacervation complexe. De plus, la structure de la sérine phosphorylée est proche de l'ostéopontine, ce qui lui confère un caractère ostéo-inducteur.

La quantité de sérine phosphorylée dans la composition peut varier et est de préférence comprise entre 20% et 50%, de préférence entre 30% et 40%, de manière plus préférée entre 32% et 35% en poids sec sur le poids total de la composition.

De préférence le ratio en poids sec céramique phosphocalcique/ sérine phosphorylée est compris entre 1,5 et 2,5, de préférence entre 1,7 et 2,3, de manière plus préférée entre 1,9 et 2.

La polydopamine, notamment sous forme nanoparticulaire (nPDA), est biocompatible et présente d'excellentes propriétés stimulatrices de l'adhésion cellulaire, de la prolifération cellulaire et de la différenciation ostéogénique. Sa structure poreuse et hydrophobe permet la formation de liaisons covalentes et hydrogènes avec l'hydroxyapatite qui est le composant principal du tissu osseux. De plus, la polydopamine induit une minéralisation apatitique en fournissant des sites nucléaires pour la minéralisation phosphocalcique dans le fluide corporel simulé (Simulated Body Fluid ou SBF en anglais).

Par « nanoparticulaire » on entend une taille de particule comprise entre 100 nm et 400 nm, de préférence entre 125 et 275 nm.

La polydopamine nanoparticulaire peut être synthétisée selon le protocole de Ju et al. Bioinspired Polymerization of Dopamine to Generate Melanin-Like Nanoparticles Having an Excellent Free-Radical-Scavenging Property. Biomacromolecules. 2011 Mar 14;12(3):625-32.

La quantité de polydopamine dans la composition peut varier et est de préférence comprise entre 1% et 5%, de préférence 2% en poids sec sur le poids total de la composition.

Selon un aspect, la céramique phosphocalcique, la sérine phosphorylée et la polydopamine sont sous forme de poudre.

Selon un mode de réalisation particulier, la polydopamine est fonctionnalisée avec au moins un principe actif choisi parmi les antibiotiques, les molécules ostéoinductrices ou peptides ostéoinducteurs, et les agents de contraste radiographiques. En effet, sa forme particulaire, et de préférence, nanoparticulaire, son caractère hydrophobe et sa réactivité à l'égard des nucléophiles, permet de contenir des principes actifs choisis parmi les antibiotiques et les molécules ostéoinductrices ou peptides ostéoinducteurs, et les agents de contraste radiographiques. Les antibiotiques permettraient de prévenir et/ou traiter les infections bactériennes qui sont une complication majeure entraînant une très grande morbidité dans les fractures. Des exemples d'antibiotiques sont la ciprofloxacine, la gentamicine, la vancomycine, la tobramycine, et la rifampicine, de préférence la ciprofloxacine. Les molécules ostéoinductrices permettraient d'accélérer l'ossification et ainsi la prise d'appui. Des exemples de molécules ostéoinductrices sont la simvastatine, les anticorps anti BMP2 (Bone Morphogenetic Protein 2). Des exemples de peptides ostéoinducteurs sont le peptide de croissance ostéogénique « Osteogenic growth peptide », le peptide de liaison aux cellules PepGen P-15, les peptides contenant le motif tripeptide Arg-Gly-Asp (RGD) « RGD containing peptide », le peptide synthétique mimétique du collagène de séquence GFOGER ou de séquence DGEA, ou encore la protéine BMP-2. Des exemples d'agents de contraste radiographiques sont des métaux tels que Cu²⁺, Mn²⁺, Fe³⁺, ou encore Gd²⁺. Lesdits métaux sont chélatés par la polydopamine et sont particulièrement adaptés pour l'IRM.

De cette manière, la composition selon l'invention présente à la fois une capacité adhésive in vivo, une biocompatibilité, mais aussi une capacité à potentialiser l'ossification osseuse ou à prévenir et/ou traiter les effets indésirables d'une fracture osseuse.

La fonctionnalisation de la polydopamine, et en particulier des nanoparticules de polydopamine, peut être faite par simples interactions hydrophobes/hydrophobes ou par interactions ioniques (liaisons non covalentes), ou par une réaction dite de Michael ou formation de base de Schiff via les fonctions amine ou thiol des peptides ou protéines (liaisons covalentes). Des exemples de fonctionnalisation de nanoparticules de polydopamine par des antibiotiques sont décrits dans Yu Fu et al. Mater. Horiz., 2021,8, 1618-1633. Des exemples de fonctionnalisation de nanoparticules de polydopamine par des molécules ostéoinductrices sont décrits dans Ko et al., Biomacromolecules, 2013, 14, 3202-3213. Des exemples de fonctionnalisation de nanoparticules de polydopamine avec du Cu²⁺ sont décrits dans Rui Ge et al. « Cu2+-Loaded Polydopamine Nanoparticles for Magnetic Resonance Imaging-Guided pH- and Near-Infrared-Light-Stimulated Thermochemotherapy" ACS Applied Materials & Interfaces 2017 9 (23), 19706-19716. Des exemples de fonctionnalisation de nanoparticules de polydopamine avec du Fe³⁺ sont décrits dans Qu J et al. "Synthesis of Biomimetic Melanin-Like Multifunctional Nanoparticles for pH Responsive Magnetic Resonance Imaging and Photothermal Therapy". Nanomaterials (Basel). 2021 Aug 19;11(8):2107. Des exemples de fonctionnalisation de nanoparticules de polydopamine avec du Gd²⁺ sont décrits dans Wang Z et al., « High Relaxivity Gadolinium-Polydopamine Nanoparticles". Small. 2017 Nov;13(43). Des exemples de fonctionnalisation de nanoparticules de polydopamine avec du Mn²⁺ sont décrits dans Dong Z et al., « Polydopamine Nanoparticles as a Versatile Molecular Loading Platform to Enable Imaging-guided Cancer Combination Therapy ». Theranostics 2016; 6(7):1031-1042.

Dans un mode de réalisation, la céramique phosphocalcique, la sérine phosphorylée et la polydopamine réagissent ensemble pour former une composition adhésive lorsqu'elles sont combinés avec un solvant aqueux. La composition peut donc comprendre en outre un solvant aqueux. Le solvant aqueux peut être de l'eau, en particulier de l'eau déminéralisée, ou une solution saline, en particulier un tampon phosphate salin, ou une solution NaCl à 0,9%. De préférence le solvant aqueux est un tampon phosphate salin.

La quantité en solvant aqueux peut varier et est de préférence comprise selon un ratio solvant/composition sèche compris entre 0,19 mL/g et 0,22 mL/g, et de préférence, selon un ratio d'environ 0,21 mL/g. Ce ratio solvant/composition sèche permet d'obtenir une masse volumique comprise entre 2 g/cm3 et 2,2 g/cm³.Dans un autre mode de réalisation, la composition adhésive selon l'invention peut en outre comprendre un additif. Cet additif peut être utilisé pour conférer une fonctionnalité supplémentaire à la composition selon l'invention, telle qu'améliorer ou affecter la manipulation, la texture, la durabilité, la résistance ou le taux de résorption du matériau, ou pour fournir des propriétés mécaniques, cosmétiques ou médicales supplémentaires. Par exemple, des polymères ou fibres comme l'acide poly(lactique-co-glycolique (PLGA) peuvent être ajoutés pour améliorer les propriétés mécaniques de la composition adhésive.

Dans un mode de réalisation, la composition adhésive permet de favoriser une nouvelle croissance osseuse au niveau du site d'application, par exemple en augmentant ou en stimulant la résorption osseuse, le dépôt ou la vitesse de remodelage.

### Procédé de préparation de la composition adhésive

Un second objet de la présente invention concerne un procédé de préparation d'une composition adhésive selon l'invention comprenant les étapes suivantes :
a) Mélanger une céramique phosphocalcique choisie parmi le tétracalcium phosphate et l'alpha-tricalcium phosphate, de préférence le tétracalcium phosphate, de la sérine phosphorylée et de la polydopamine dans un récipient,
b) Ajouter un solvant à la précédente préparation,
c) Récupérer le mélange ainsi formé.

De préférence, la céramique phosphocalcique, la sérine phosphorylée et la polydopamine sont sous forme de poudre. Selon un autre aspect, la polydopamine est sous forme de poudre nanoparticulaire.

Selon un aspect, la masse volumique de la composition obtenue est comprise entre 2 g/cm3 et 2,2 g/cm3, cette masse volumique est obtenue notamment en respectant un ratio liquide/poudre compris entre 0,19 mL/g et 0,22 mL/g, et de préférence un ratio d'environ 0,21 mL/g.

Le mélange ainsi formé peut se présenter sous forme de fluide ou semi-solide tel qu'une pâte, de préférence sous forme de pâte. Cette pâte pourra ensuite être directement utilisée par le praticien qualifié sur le sujet humain ou animal.

Typiquement, une composition adhésive obtenue présente un temps de prise ou de durcissement initial compris entre 2 et 3 minutes, et un temps de prise ou durcissement final compris entre 4 et 8 minutes. Ce temps de prise permet avantageusement au praticien qualifié de pouvoir l'utiliser lors de chirurgie et de pouvoir attendre la prise finale avant de refermer le sujet.

Dans tous les modes de réalisation, un praticien qualifié, par exemple, un médecin, un dentiste, un chirurgien, une infirmière ou une autre personne appropriée peuvent modifier les composants spécifiques pour obtenir les propriétés adhésives souhaitées de ladite composition en fonction de l'utilisation prévue ou du résultat souhaité.

### Utilisation de la composition adhésive

La composition adhésive peut être utilisée dans une grande variété d'applications. Un objet de la présente invention concerne la composition selon l'invention pour son utilisation thérapeutique chez l'homme ou l'animal, de préférence pour son utilisation en procédure médicale notamment lors de chirurgie du tissu osseux, et plus préférentiellement, pour son utilisation *in vivo* comme adhésif osseux. La chirurgie du tissu osseux peut être une chirurgie dentaire, des sinus, de la face ou autre région squelettique.

Par exemple, la composition adhésive peut être utilisée pour faire adhérer une structure à une surface.

Par « structure » on entend un objet solide. La structure peut être un os ou autre os ou fragment d'os, un implant, une greffe, un dispositif, ou des tissus biologiques. Des exemples de tissus biologiques peuvent être un tendon ou un ligament, typiquement le ligament croisé antérieur ou postérieur.

Par « surface » on entend une surface biologique. Cette surface biologique peut être celle d'un os et notamment, le périoste, fine enveloppe conjonctive enveloppant l'os qu'aux surfaces non recouvertes par du cartilage, ou l'os compact, partie périphérique et dense de l'os, ou encore l'os spongieux, partie centrale de l'os, ou alors celle d'un tendon ou d'un ligament.

Dans un mode de réalisation, la surface est préparée afin de recevoir la composition adhésive, par exemple en avivant la surface de l'os.

Dans un mode de réalisation l'adhérence de la structure à la surface de par l'application de la composition adhésive, est permanente ou destinée à être permanente ou jusqu'à ce que la composition adhésive soit résorbée ou remplacée par de l'os.

Dans un mode de réalisation, la composition adhésive est utilisée pour combler un espace, trou ou vide dans ladite surface, soit avant ou après le placement de ladite structure.

Dans certains modes de réalisation, les compositions adhésives sont utilisées pour réparer un défaut dans un os causé par une maladie ou un état, comme le cancer (par exemple, l'ostéosarcome), l'ostéoporose, le rachitisme, une tumeur maligne de l'os, une infection de l'os ou une autre maladie génétique ou développementale.

Dans certains modes de réalisation, les compositions adhésives sont utilisées pour renforcer un os chez un sujet qui a été affaibli par une maladie ou un état, comme le cancer (par exemple, l'ostéosarcome), l'ostéoporose, le rachitisme, une tumeur maligne de l'os, une infection de l'os ou une autre maladie génétique ou développementale.

Dans certains modes de réalisation, le sujet a subi un traumatisme, tel qu'un os cassé, un os fracturé, un ligament rompu, un tendon rompu ou une dent endommagée. Typiquement, la composition adhésive peut permettre de réparer le ligament rompu ou le tendon rompu, par exemple lors de rupture du ligament croisé pour assurer à nouveau la mobilité de l'articulation du genou.

Dans certains modes de réalisation, le sujet subit une intervention de chirurgie plastique ou reconstructrice.

Les compositions et les procédés peuvent être utilisés pour traiter un sujet souffrant ou affligé de toute maladie ou condition qui a un impact sur l'intégrité structurelle du squelette osseux ou du tissu conjonctif fibreux.

Selon un autre aspect, la composition adhésive selon l'invention peut être utilisée ex vivo.

### Kit

Un objet de la présente invention concerne un kit pour préparer une composition adhésive selon l'invention, comprenant :
- une céramique phosphocalcique choisie parmi le tétracalcium phosphate et l'alpha-tricalcium phosphate, de préférence le tétracalcium phosphate,
- de la sérine phosphorylée, et
- de la polydopamine.

Typiquement chaque composant est sous forme de poudre, de préférence la polydopamine est sous forme de poudre nanoparticulaire.

Chaque composant peut être conditionné sous forme séparée, ou un composant peut être séparé dans son contenant et les deux autres composants peuvent être mélangés dans un même contenant, ou tous les composants sont conditionnés ensemble dans un même contenant.

Dans un mode de réalisation, le kit comprend en outre un solvant aqueux. Dans ce cas, le solvant aqueux peut être dans un contenant séparé et les autres composants, notamment sous forme de poudre, peuvent être mélangés dans un même contenant ou séparés chacun dans des contenants différents ou un composant est séparé dans un contenant et les deux autres composants sont mélangés dans un contenant différent. De préférence, les contenants utilisés sont scellés conformément aux bonnes pratiques d'emballage pour préserver la durée de conservation des composants mélangés ou séparés. Dans certains modes de réalisation, la préservation de la durée de conservation des composants dans le kit comprend le maintien de la stérilité. Si des additifs sont inclus dans ledit kit, ils peuvent être mélangés à l'un ou à la totalité des composant ou présent dans un contenant séparé.

Ledit kit peut comprendre des composants supplémentaires pour la préparation ou l'application de la composition adhésive selon l'invention, tels que des bols ou des surfaces de mélange, des bâtonnets d'agitation, des seringues, des cathéters adaptés aux seringues, par exemple des cathéters présentant une grande taille de gauge, typiquement 10G, des spatules, des seringues, des pistolets thermiques par rayonnements UV ou infrarouges ou d'autres dispositifs de préparation ou de distribution.

### BREVE DESCRIPTION DES DESSINS

**Fig. 1**

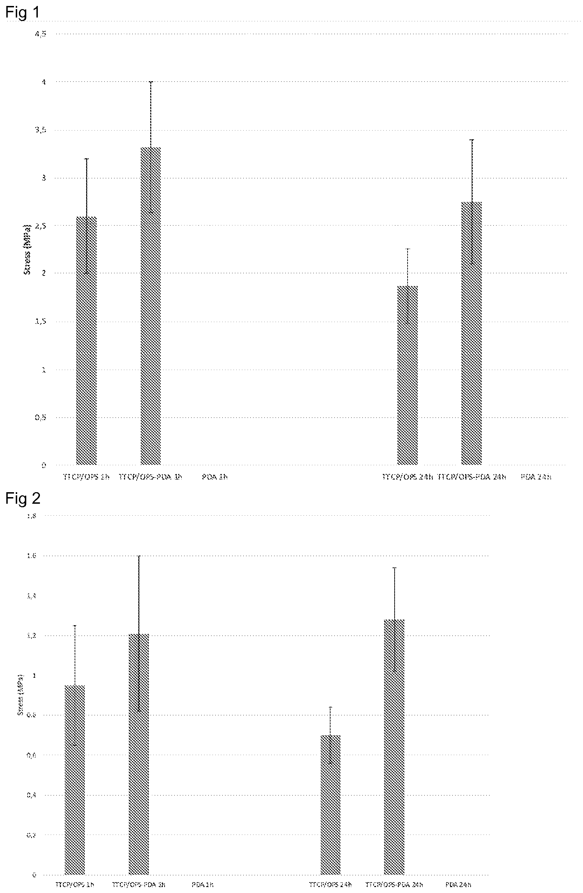

   [Fig. 1] Résultats des tests de traction mécanique sur titane. Contrainte maximale (MPa) moyenne des échantillons collés (n=8). * signifie différence significative (p=0,0029).
**Fig. 2**
   [Fig. 2] Résultats des tests de traction mécanique sur os bovins. Contrainte maximale (MPa) moyenne des échantillons collés (n=8). * signifie différence significative (p=0,00093).
**Fig. 3**

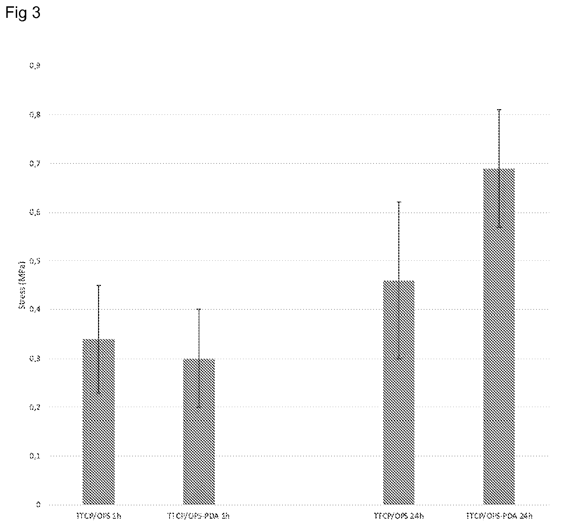

   [Fig. 3] Résultats des tests de traction ex vivo des compositions adhésives selon l'exemple 1. Contrainte maximale (MPa) moyenne des échantillons collés (n=7). * signifie différence significative (p=0,029).

### EXEMPLES

### Exemple 1 : Préparation de la composition adhésive selon l'invention

### Synthèse polydopamine nanoparticulaire (nPDA)

900 mg d'hydrochloride de dopamine a été dissous dans 450 mL d'eau pure. La solution a été chauffée à 50°C et 3,8 mL d'hydroxyde de sodium a été ajouté. La solution a été maintenue à 50°C pendant 5 h. La solution a alors eté dialysée à l'aide de membranes Spectra/Por 6 (Spectrum Labs, Repligen, Waltham, MA, Etats-Unis) afin d'obtenir des nPDA en suspension. Les nPDA en poudre ont été obtenues après lyophilisation de cette solution.

### Préparation de la composition

183mg de poudre de tétracalcium phosphate (TTCP) de chez Matexcel, avec 92,5mg de poudre de O-Phospho-DL-Sérine de chez Merck (OPS) et 5,5mg de poudre nanoparticulaire de polydopamine (nPDA), telle qu'obtenue ci-dessus, ont été versées dans un mortier où elles sont mélangées à la spatule puis au pilon afin d'obtenir un mélange homogène. 59µL de tampon phosphate salin (PBS, pH 7,4 de chez Merck) ont été ajouté à la micropipette selon un ratio liquide/poudre de 0,21 mL/g. Les poudres et le tampon phosphate salin ont été mélangés pendant 10 secondes à l'aide d'une spatule afin d'obtenir une pâte. La composition adhésive obtenue est la composition selon l'invention.

Sans vouloir être lié par une quelconque théorie, le tétracalcium phosphate forme des interactions ioniques avec la sérine phosphorylée, qui, lorsqu'ils sont combinés dans certains rapports et avec de la polydopamine, réagissent pour fournir un matériau ayant des capacités adhésives avantageuses par rapport à une composition ne contenant que du tétracalcium phosphate et de la sérine phosphorylée. En effet, le tétracalcium phosphate subit une réaction de dissolution-précipitation en hydroxyapatite de façon spontanée. De par l'ajout de sérine phosphorylée, cette réaction est empêchée et entraîne la formation de monohydrate de phosphosérine calcique qui forme un réseau coordonné permettant l'adhésion aux tissus osseux et l'initiation de la calcification osseuse. La polydopamine, de par ses propriétés adhésives et d'induction ostéogéniques, potentialise l'effet adhésif en favorisant la reconstruction osseuse.

La même préparation a été réalisée cette fois-ci sans ajout de poudre nanoparticulaire de polydopamine. Cette préparation est une composition comparative telle qu'on peut la retrouver dans l'art antérieur.

### Exemple 2 : Tests de traction mécanique sur titane et os bovins de la composition selon l'invention

Des échantillons métalliques (titane) et osseux (os bovins) ont été utilisés. Les échantillons métalliques étaient constitués par des cylindres de titane avec une surface de collage de 200 mm². Les échantillons osseux étaient constitués par des parallélépipèdes rectangles appariés avec une surface de collage de 100 à 180 mm².

Les compositions adhésives préparées à l'exemple 1 ou une suspension de nanoparticules de polydopamine telle qu'obtenue dans l'exemple 1, ont été appliquées en couche fine sur les surfaces des échantillons (N=8). Une compression manuelle des échantillons entre eux a été réalisée et maintenue pendant 4 minutes. Les échantillons collés étaient ensuite immergés pendant 1 heure ou 24 heures dans un bain de PBS à 37°C permettant de simuler l'environnement aqueux physiologique.

Ensuite, l'évaluation mécanique de la force d'adhésion était réalisée en traction (formation bout à bout) jusqu'à rupture sur une machine Instron 4466 (Norwood, MA, Etats-Unis). La cellule de charge était de 1000 N lors des tests avec les échantillons d'os et de 10000 N lors des tests avec les échantillons en titane. La vitesse de déplacement était de 0,1 mm/s.

Les résultats ont été décrits sous forme de contrainte de traction, soit la force de rupture par unité de surface appliquée aux échantillons collés. Les résultats ont été analysés statistiquement de façon non paramétrique par test de Mann-Whitney.

Les résultats pour les échantillons de titane (voir Fig 1) démontrent que l'adhésion était significativement supérieure avec la composition selon l'invention (TTCP/OPS-nPDA) par rapport à la composition comparative (TTCP/OPS) et à la suspension de nanoparticules de polydopamine à 24h (p=0,0029).

Les résultats pour les échantillons d'os bovins (voir Fig 2) démontrent que l'adhésion était significativement supérieure avec la composition selon l'invention (TTCP/OPS-nPDA) par rapport à la composition comparative (TTCP/OPS) et à la suspension de nanoparticules de polydopamine à 24h (p=0,00093).

### Exemple 3 : Tests de traction ex vivo de la composition selon l'invention

Le test suivant a été conçu pour être plus proche d'une situation clinique en imitant la réalisation d'une autogreffe osseuse. Des échantillons de tibias et de fragments de fibulas (6 mm de long pour 1 mm de large) de rat fraîchement sacrifié ont été utilisés. Le tibia était légèrement avivé sur sa face externe, de façon similaire à ce qui est réalisé avant la mise en place d'une autogreffe osseuse.

Les compositions adhésives préparées à l'exemple 1, avec et sans nPDA, étaient appliquées en couche fine sur le tibia (N=7). Un fil de Vicryl était apposé puis le fragment de fibula était mis en place. Une compression des échantillons entre eux était réalisée et maintenue pendant 4 minutes. Les échantillons collés étaient ensuite immergés pendant 1 heure ou 24 heures dans un bain de PBS à 37°C permettant de simuler l'environnement aqueux physiologique.

Ensuite, l'évaluation mécanique de la force d'adhésion était réalisée en traction par la mise en place de poids standardisés de masse croissante sur le fil de Vicryl jusqu'à rupture (Figure 2).

Les résultats ont été décrits sous forme de contrainte de traction, soit la force de rupture par unité de surface appliquée aux échantillons collés. Les résultats ont été analysés statistiquement de façon non paramétrique par test de Mann-Whitney.

Les résultats sur les échantillons de tibias et de fragments de fibulas (voir Fig. 3) démontrent que l'adhésion était significativement supérieure avec la composition selon l'invention (TTCP/OPS-nPDA) par rapport à la composition comparative (TTCP/OPS) à 24h (p=0,029).

## Revendications

1. Composition adhésive comprenant :
- une céramique phosphocalcique choisie parmi le tétracalcium phosphate et l'alpha-tricalcium phosphate, de préférence le tétracalcium phosphate,
- de la sérine phosphorylée,
- de la polydopamine, et
- un solvant aqueux.

2. Composition selon la revendication 1, dans laquelle la quantité de céramique phosphocalcique est comprise entre 50% et 80%, de préférence entre 60% et 70%, de manière plus préférée entre 65 et 68% en poids sec.

3. Composition selon la revendication 1 ou 2, dans laquelle la quantité de sérine phosphorylée est comprise entre 20% et 50%, de préférence entre 30% et 40%, de manière plus préférée entre 32% et 35% en poids sec.

4. Composition selon les revendications 1 à 3, dans laquelle le ratio en poids sec céramique phosphocalcique/ sérine phosphorylée est compris entre 1,5 et 2,5, de préférence entre 1,7 et 2,3, de manière plus préférée entre 1,9 et 2.

5. Composition selon les revendications 1 à 4, dans laquelle la quantité de polydopamine est comprise entre 1% et 5%, de préférence 2% en poids sec.

6. Composition selon les revendications 1 à 5, dans laquelle la masse volumique de la composition est comprise entre 2 g/cm3 et 2,2 g/cm3.

7. Composition selon les revendications 1 à 6, dans laquelle la polydopamine est fonctionnalisée avec au moins un principe actif choisi parmi les antibiotiques, les molécules ostéoinductrices et les agents de contraste radiographiques.

8. Kit pour préparer une composition adhésive selon les revendications 1 à 7, comprenant :
- une céramique phosphocalcique choisie parmi le tétracalcium phosphate et l'alpha-tricalcium phosphate, de préférence le tétracalcium phosphate,
- de la sérine phosphorylée, et
- de la polydopamine.

9. Kit selon la revendication 8, dans lequel la céramique phosphocalcique, la sérine phosphorylée et la polydopamine sont sous forme de poudre, de préférence la polydopamine est sous forme de poudre nanoparticulaire.

10. Procédé de préparation d'une composition adhésive selon les revendications 1 à 7 comprenant les étapes suivantes :
a) Mélanger une céramique phosphocalcique choisie parmi le tétracalcium phosphate et l'alpha-tricalcium phosphate, de préférence le tétracalcium phosphate, de la sérine phosphorylée et de la polydopamine dans un récipient,
b) Ajouter un solvant à la précédente préparation,
c) Récupérer le mélange ainsi formé.

11. Procédé selon la revendication 10, dans lequel la céramique phosphocalcique, la sérine phosphorylée et la polydopamine sont sous forme de poudre, de préférence la polydopamine est sous forme de poudre nanoparticulaire.

12. Composition adhésive selon les revendications 1 à 7, pour son utilisation thérapeutique *in vivo* comme adhésif osseux.

## Patentansprüche

1. Klebstoffzusammensetzung, umfassend:
- eine Phosphocalciumkeramik, gewählt aus Tetracalciumphosphat und Alphatricalciumphosphat, bevorzugt Tetracalciumphosphat,
- phosphoryliertes Serin,
- Polydopamin und
- ein wässriges Lösungsmittel.

2. Zusammensetzung nach Anspruch 1, wobei die Menge an Phosphocalciumkeramik zwischen 50 % und 80 %, bevorzugt zwischen 60 % und 70 %, stärker bevorzugt zwischen 65 % und 68 % der Trockenmasse beträgt.

3. Zusammensetzung nach Anspruch 1 oder 2, wobei die Menge an phosphoryliertem Serin zwischen 20 % und 50 %, bevorzugt zwischen 30 % und 40 %, stärker bevorzugt zwischen 32 % und 35 % der Trockenmasse beträgt.

4. Zusammensetzung nach den Ansprüchen 1 bis 3, wobei das Verhältnis von Phosphocalciumkeramik zu phosphoryliertem Serin in der Trockenmasse zwischen 1,5 und 2,5, bevorzugt zwischen 1,7 und 2,3, stärker bevorzugt zwischen 1,9 und 2 liegt.

5. Zusammensetzung nach den Ansprüchen 1 bis 4, wobei die Menge an Polydopamin zwischen 1 % und 5 %, bevorzugt 2 % der Trockenmasse beträgt.

6. Zusammensetzung nach den Ansprüchen 1 bis 5, wobei die Volumenmasse der Zusammensetzung zwischen 2 g/cm3 und 2,2 g/cm3 liegt.

7. Zusammensetzung nach den Ansprüchen 1 bis 6, wobei das Polydopamin mit wenigstens einem Wirkstoff funktionalisiert ist, der aus Antibiotika, osteoinduktiven Molekülen und Röntgenkontrastmitteln gewählt ist.

8. Kit zur Herstellung einer adhäsiven Zusammensetzung gemäß den Ansprüchen 1 bis 7, umfassend:
- eine Phosphocalciumkeramik, ausgewählt aus Tetracalciumphosphat und Alphatricalciumphosphat, bevorzugt Tetracalciumphosphat,
- phosphoryliertes Serin und
- Polydopamin.

9. Kit nach Anspruch 8, wobei die Phosphocalciumkeramik, das phosphorylierte Serin und das Polydopamin in Pulverform vorliegen und bevorzugt das Polydopamin in nanopartikulärer Pulverform vorliegt.

10. Verfahren zur Herstellung einer Klebstoffzusammensetzung nach den Ansprüchen 1 bis 7, umfassend die folgenden Schritte:
a) Mischen einer Phosphocalciumkeramik, gewählt aus Tetracalciumphosphat und Alpha-Tricalciumphosphat, bevorzugt Tetracalciumphosphat, phosphoryliertem Serin und Polydopamin in einem Behälter,
b) Hinzufügen eines Lösungsmittels zu der vorherigen Zubereitung,
c) Gewinnen der so gebildeten Mischung.

11. Verfahren nach Anspruch 10, wobei die Phosphocalciumkeramik, das phosphorylierte Serin und das Polydopamin in Pulverform vorliegen, bevorzugt das Polydopamin in nanopartikulärer Pulverform vorliegt.

12. Klebstoffzusammensetzung nach den Ansprüchen 1 bis 7 zur therapeutischen Verwendung *in vivo* als Knochenklebstoff.

## Claims

1. Adhesive composition comprising:
- a calcium phosphate ceramic selected from among tetracalcium phosphate and alpha-tricalcium phosphate, preferably tetracalcium phosphate,
- phosphorylated serine,
- polydopamine, and
- an aqueous solvent.

2. The composition according to claim 1, wherein the amount of calcium phosphate ceramic is comprised between 50% and 80%, preferably between 60% and 70%, more preferably between 65 and 68% by dry weight.

3. The composition according to claim 1 or 2, wherein the amount of phosphorylated serine is comprised between 20% and 50%, preferably between 30% and 40%, more preferably between 32% and 35% by dry weight.

4. The composition according to claims 1 to 3, wherein the calcium phosphate ceramic /phosphorylated serine dry weight ratio is comprised between 1.5 and 2.5, preferably between 1.7 and 2.3, more preferably between 1.9 and 2.

5. The composition according to claims 1 to 4, wherein the amount of polydopamine is comprised between 1% and 5%, preferably 2% by dry weight.

6. The composition according to claims 1 to 5, wherein the volumetric mass of the composition is comprised between 2 g/cm3 and 2.2 g/cm3.

7. The composition according to claims 1 to 6, wherein the polydopamine is functionalised with at least one active ingredient selected from antibiotics, osteoinductive molecules and x-ray contrast agents.

8. A kit for preparing an adhesive composition according to claims 1 to 7, comprising:
- a calcium phosphate ceramic selected from among tetracalcium phosphate and alpha-tricalcium phosphate, preferably tetracalcium phosphate,
- phosphorylated serine, and
- polydopamine.

9. The kit according to claim 8, wherein the calcium phosphate ceramic, the phosphorylated serine and the polydopamine are in powder form, preferably the polydopamine is in the form of nanoparticle powder.

10. Method for preparing an adhesive composition according to claims 1 to 7, comprising the following steps:
a) Mixing a calcium phosphate ceramic selected from among tetracalcium phosphate and alpha-tricalcium phosphate, preferably tetracalcium phosphate, phosphorylated serine and polydopamine in a container,
b) Adding a solvent to the previous preparation,
c) Recovering the mixture thus formed.

11. The method according to claim 10, wherein the calcium phosphate ceramic, the phosphorylated serine and the polydopamine are in powder form, preferably the polydopamine is in the form of nanoparticle powder.

12. The adhesive composition according to claims 1 to 7, for the therapeutic use thereof *in vivo* as a bone adhesive.
